# EUROPEAN PATENT SPECIFICATION

(11) **EP 3 897 759 B1**
(45) Date of publication and mention of the grant of the patent: **13.11.2024**
(21) Application number: 19839495.9
(22) Date of filing: 17.12.2019
(51) Int. Cl.: A61F 13/00, A61F 17/00

(54) **WOUND DRESSINGS INCLUDING PVP-CITRIC ACID COPOLYMER**
WUNDVERBÄNDE MIT PVP-ZITRONENSÄURE-COPOLYMER
PANSEMENTS COMPRENANT UN COPOLYMÈRE PVP-ACIDE CITRIQUE

(30) Priority: 21.12.2018 US 201862783786 P
(43) Date of publication of application: 27.10.2021
(73) Proprietor: 3M Innovative Properties Company, Saint Paul, MN 55133-3427 (US); Systagenix Wound Management, Limited, Gatwick Airport, West Sussex RH6 0PA (GB)
(72) Inventor: LOCKE, Christopher B., San Antonio, Texas 78265 (US); BOURDILLON, Katherine A., West Sussex RH6 0PA (GB); WHAITES, Adam, West Sussex RH6 OPA (GB); WAITE, Alexander, West Sussex RH6 OPA (GB)
(74) Representative: Simmons & Simmons
(86) International application number: PCT/US2019/066744
(87) International publication number: WO 2020/131806

(56) References cited:
- EP-A1- 0 918 548
- WO-A1-2004/080500
- WO-A1-2016/120620
- WO-A1-2018/035063
- US-A1- 2013 052 257
- AJJI ET AL: "Grafting of poly(vinyl pyrrolidone) with citric acid using gamma irradiation", NUCLEAR INSTRUMENTS & METHODS IN PHYSICS RESEARCH. SECTION B: BEAM INTERACTIONS WITH MATERIALS AND ATOMS, ELSEVIER BV, NL, vol. 265, no. 1, 17 November 2007 (2007-11-17), pages 179 - 182, XP022349407, ISSN: 0168-583X

## Description

### TECHNICAL FIELD

This application claims the benefit of and priority to U.S. Provisional Application No. 62/783,786, filed December 21, 2018.

### TECHNICAL FIELD

The present technology relates generally to dressings useful for treating wounds that include a co-polymer of polyvinylpyrrolidone (PVP) and citric acid. Such dressings may be used to disrupt and/or prevent biofilm formation in a wound upon application.

### BACKGROUND

The following description of the background of the present technology is provided simply as an aid in understanding the present technology and is not admitted to describe or constitute prior art to the present technology.

Infections can retard wound healing and, if untreated, can result in tissue loss, systemic infections, septic shock, and death. Moreover, in addition to vegetative or free-floating bacteria present in a wound, bacterial biofilms may also form in a wound presenting further challenges in wound therapy, particularly chronic wounds. A biofilm comprises a polysaccharide extracellular matrix produced by an association of microorganisms (e.g., single or multiple species) that have adhered onto a surface, forming three-dimensional microbial communities. The ability of bacteria to form these complex biofilms can impede a host's defense mechanisms against pathogens. Currently, there is an unmet need for wound dressing products that can prevent, reduce, inhibit, or disrupt biofilm formation in a wound upon application, and over time.

Document US 2013/052257 A1 discloses a wound dressing comprising a PVP hydrogel, the addition of active agents makes it suitable to inhibit gram negative microorganisms and gram-positive microorganism.

### SUMMARY

In an aspect, a dressing composition is provided that includes a hydrogel, wherein the hydrogel includes a co-polymer of polyvinylpyrrolidone (PVP) and citric acid. The wound dressing may include a first layer that includes the hydrogel and a second layer that includes a mixture of a collagen and an oxidized regenerated cellulose (ORC).

In a related aspect, a kit is provided that includes a dressing composition of any embodiment described herein and instructions for use.

In another aspect, a wound dressing composition is provided that includes a mixture of a collagen, an oxidized regenerated cellulose (ORC), and a co-polymer of polyvinylpyrrolidone (PVP) and citric acid.

In a related aspect, a kit is provided that includes a wound dressing composition of any embodiment described herein and instructions for use.

### DETAILED DESCRIPTION

It is to be appreciated that certain aspects, modes, embodiments, variations and features of the present methods are described below in various levels of detail in order to provide a substantial understanding of the present technology.

### Definitions

The definitions of certain terms as used in this specification are provided below. Unless defined otherwise, all technical and scientific terms used herein generally have the same meaning as commonly understood by one of ordinary skill in the art to which this present technology belongs.

The following terms are used throughout as defined below.

As used herein and in the appended claims, singular articles such as "a", "an", and "the" and similar referents in the context of describing the elements (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e.g.,* "such as") provided herein, is intended merely to better illuminate the embodiments and does not pose a limitation on the scope of the claims unless otherwise stated. No language in the specification should be construed as indicating any non-claimed element as essential.

As used herein, "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 10% of the particular term - for example, "about 10 wt.%" would be understood to mean "9 wt.% to 11 wt.%." It is to be understood that when "about" precedes a term, the term is to be construed as disclosing "about" the term as well as the term without modification by "about" - for example, "about 10 wt.%" discloses "9 wt.% to 11 wt.%" as well as disclosing "10 wt.%."

As used herein, the terms "moisture vapor transmission rate" and "MVTR" will be understood by persons of ordinary skill in the art as a measure of the passage of water vapor through a substance of a given unit area and unit time. The most common international unit for the MVTR is g/m²/day, wherein 1 day = 24 hr.

As understood by one of ordinary skill in the art, "molecular weight" (also known as "relative molar mass") is a dimensionless quantity but is converted to molar mass by multiplying by 1 gram/mole - for example, collagen with a weight-average molecular weight of 5,000 has a weight-average molar mass of 5,000 g/mol.

As used herein, the term "dressing(s)"includes the dressing compositions, the wound dressing compositions, and the wound dressings of the present technology.

As used herein, the term "solid content" refers to the density of a material and/or film of the wound dressing of the present technology, which is its mass per unit volume.

The term "mammalian recombinant collagen" refers to collagen manufactured by culturing a non-human organism or mammalian or non-mammalian cells to express at least one exogenous gene encoding a collagen in the culturing system. The collagen may be recombinantly manufactured by a plant (*e.g.,* CollPlant, CollPlant Holdings Ltd., Ness Ziona, Israel) such as tobacco, or in yeast. The term "human recombinant collagen" refers to collagen manufactured by culturing a non-human organism or mammalian or non-mammalian cells to express at least one human gene encoding a collagen. The human recombinant collagen may be selected from the group consisting of collagen type I, type II, type III, type IV, type V, type VI, type VII, type VIII, type IX, type X, type XI, type XII, type XIII, type XIV, type XV, type XVI, type XVII, type XVIII, type XIX, type XX, type XXI, type XXII, type XXIII, type XXIV, type XXV, type XXVI, and type XXVII. The human recombinant collagen can be collagen of one type free of any other type, or can be a mixture of collagen types. Suitably, the human recombinant collagen comprises collagens selected from the group consisting of collagen type I, collagen type III, and mixtures thereof. The term "bovine recombinant collagen" refers to collagen manufactured by culturing a non-human organism or mammalian or non-mammalian cells to express at least one bovine gene encoding a collagen. The bovine recombinant collagen may be selected from the group consisting of collagen type I, type II, type III, and type IV. The bovine recombinant collagen can be collagen of one type free of any other type, or can be a mixture of collagen types. Suitably, the bovine recombinant collagen comprises collagens selected from the group consisting of collagen type I, collagen type III, and mixtures thereof.

As used herein, the term "biofilm" refers to an extracellular matrix created by an association of microorganisms, *e.g.*, single or multiple species. The microorganisms can be encased or embedded in a matrix material, which may be self-produced by resident microorganisms. The biofilm may be present or adhere to living and/or non-living surfaces, *e.g.*, tissue, a wound, medical implants, including but not limited to, orthopedic implants, dental implants, catheters, stents and so on. Exemplary microorganisms include, but are not limited to bacteria, *e.g.*, Gram-negative bacteria, such as *Pseudomonas aeruginosa,* Gram-positive bacteria, such as *Staphylococcus aureus* and *Streptococcus mutans,* and fungi, such as yeasts, *e.g., Candida albicans.* The term "matrix material" is intended to encompass extracellular polymeric substances. Exemplary matrix materials include, but are not limited to polysaccharides, glycoproteins and/or nucleic acids. The term "biofilm" is further intended to include biological films that develop and persist at interfaces in aqueous environments. The language "biofilm development" or "biofilm formation" is intended to include the formation, growth, and modification of the bacterial colonies contained with biofilm structures, as well as the synthesis and maintenance of the exopolysaccharide of the biofilm structures. "Reducing" or "disrupting" a biofilm includes reducing the number of total viable microorganisms making up and/or embedded in at least part of the biofilm, for example, as measured by total viable counts (TVC) of microorganisms (e.g., bacteria, yeast).

As used herein, the term "% (w/v)" refers to the percent of weight of the solute in the total volume of the solution, *i.e.*, the number of grams of solute in 100 mL of solution.

As used herein, pharmaceutically acceptable salts of compounds described herein are within the scope of the present technology and include acid or base addition salts which retain the desired pharmacological activity and is not biologically undesirable (e.g., the salt is not unduly toxic, allergenic, or irritating, and is bioavailable). When the compound of the present technology has a basic group, such as, for example, an amino group, pharmaceutically acceptable salts can be formed with inorganic acids (such as hydrochloric acid, hydroboric acid, nitric acid, sulfuric acid, and phosphoric acid), organic acids (e.g., alginate, formic acid, acetic acid, benzoic acid, gluconic acid, fumaric acid, oxalic acid, tartaric acid, lactic acid, maleic acid, citric acid, succinic acid, malic acid, methanesulfonic acid, benzenesulfonic acid, naphthalene sulfonic acid, and p-toluenesulfonic acid) or acidic amino acids (such as aspartic acid and glutamic acid). When the compound of the present technology has an acidic group, such as for example, a carboxylic acid group, it can form salts with metals, such as alkali and earth alkali metals (e.g., Na⁺, Li⁺, K⁺, Ca²⁺, Mg²⁺, Zn²⁺), ammonia or organic amines (e.g. dicyclohexylamine, trimethylamine, triethylamine, pyridine, picoline, ethanolamine, diethanolamine, triethanolamine) or basic amino acids (e.g., arginine, lysine and ornithine). Such salts can be prepared in situ during isolation and purification of the compounds or by separately reacting the purified compound in its free base or free acid form with a suitable acid or base, respectively, and isolating the salt thus formed.

As used herein, the "administration" of a dressing to a subject includes any route of introducing or delivering to a subject a dressing to perform its intended function. Administration can be carried out by any suitable route, including but not limited to, topical administration. Administration includes self-administration and the administration by another.

As used herein, the term "effective amount" refers to a quantity sufficient to achieve a desired therapeutic and/or prophylactic effect, *e.g.*, an amount which results in the decrease in a wound described herein or one or more signs or symptoms associated with a wound described herein. In the context of therapeutic or prophylactic applications, the amount of a dressing administered to the subject will vary depending on the composition, the degree, type, and severity of the wound and on the characteristics of the individual. The dressings can also be administered in combination with one or more additional therapeutic compounds. In the methods described herein, the therapeutic dressings may be administered to a subject having one or more wounds.

As used herein, the terms "individual", "patient", or "subject" can be an individual organism, a vertebrate, a mammal, or a human. In some embodiments, the individual, patient or subject is a human.

"Treating" or "treatment" as used herein covers the treatment of a wound described herein, in a subject, such as a human, and includes: (i) inhibiting a wound, i.e., arresting its development; (ii) relieving a wound, i.e., causing regression of the wound; (iii) slowing progression of the wound; and/or (iv) inhibiting, relieving, or slowing progression of one or more symptoms of the wound. In some embodiments, treatment means that the symptoms associated with the wound are, *e.g.*, alleviated, reduced, cured, or placed in a state of remission.

It is also to be appreciated that the various modes of treatment of wounds as described herein are not part of the invention, and are intended to mean "substantial," which includes total but also less than total treatment, and wherein some biologically or medically relevant result is achieved. The treatment may be a continuous prolonged treatment for a chronic wound or a single, or few time administrations for the treatment of an acute wound.

### The Dressings of the Present Technology

Wounds are typically contaminated by bacteria, however when the immune system cannot cope with normal bacterial growth, a wound can become infected. An infected wound is a wound in which bacteria or other microorganisms have colonized, causing a deterioration and delay in the healing of the wound. Thus, a reduction in bacterial colonization is vital in wound therapy.

A biofilm consists of a polysaccharide extracellular matrix produced by an association of microorganisms *(e.g.,* single or multiple species) that have adhered onto a surface. These three-dimensional microbial communities can have coordinated multi-cellular behavior, thereby forming an extracellular matrix in which the bacteria are embedded. The ability of bacteria to form these complex biofilms can impede a host's defense mechanisms against pathogens. As such, biofilms often display a heightened tolerance to antimicrobial treatment.

The present disclosure is directed to dressings that which may prevent, reduce, inhibit, or disrupt biofilm levels in a wound upon application, and over time.

### The Dressing Composition

In an aspect, the present disclosure provides a dressing composition that includes a hydrogel, where the hydrogel includes a co-polymer of polyvinylpyrrolidone (PVP) and citric acid (PVP-CA). The hydrogel also includes water. The co-polymer may be included in the hydrogel at about 5 % (w/v) to about 25 % (w/v) based on the total volume of the hydrogel. Thus, the co-polymer may be included in the hydrogel at about 5 % (w/v), about 5.2 % (w/v), about 5.4 % (w/v), about 5.6 % (w/v), about 5.8 % (w/v), about 6 % (w/v), about 6.2 % (w/v), about 6.4 % (w/v), about 6.6 % (w/v), about 6.8 % (w/v), about 7 % (w/v), about 7.2 % (w/v), about 7.4 % (w/v), about 7.6 % (w/v), about 7.8 % (w/v), about 8 % (w/v), about 8.2 % (w/v), about 8.4 % (w/v), about 8.6 % (w/v), about 8.8 % (w/v), about 9 % (w/v), about 9.2 % (w/v), about 9.4 % (w/v), about 9.6 % (w/v), about 9.8 % (w/v), about 10 % (w/v), about 10.5 % (w/v), about 11 % (w/v), about 11.5 % (w/v), about 12 % (w/v), about 12.5 % (w/v), about 13 % (w/v), about 13.5 % (w/v), about 14 % (w/v), about 14.5 % (w/v), about 15 % (w/v), about 15.5 % (w/v), about 16 % (w/v), about 16.5 % (w/v), about 17 % (w/v), about 17.5 % (w/v), about 18 % (w/v), about 18.5 % (w/v), about 19 % (w/v), about 19.5 % (w/v), about 20 % (w/v), about 20.5 % (w/v), about 21 % (w/v), about 21.5 % (w/v), about 22 % (w/v), about 22.5 % (w/v), about 23 % (w/v), about 23.5 % (w/v), about 24 % (w/v), about 24.5 % (w/v), about 25 % (w/v), or any range including and/or in between any two of these values.

The copolymer of any embodiment described herein may include a weight ratio of PVP to citric acid of about 25:1 to about 1:2. Thus, in any embodiment described herein, the weight ratio of PVP to citric acid in the co-polymer may be about 25:1, about 24:1, about 23:1, about 22:1, about 21:1, about 20:1, about 19:1, about 18:1, about 17:1, about 16:1, about 15:1, about 14:1, about 13:1, about 12:1, about 11:1, about 10:1, about 9:1, about 8:1, about 7:1, about 6:1, about 5:1, about 4:1, about 3:1, about 2:1, about 1:1, about 1:2, or any range including and/or in between any two of these values.

In any embodiment disclosed herein, the hydrogel may include a wound-facing side and an environmental-facing side. The wound-facing side of the hydrogel may be configured to be in contact with a wound when in use.

In any embodiment disclosed herein, the thickness of the hydrogel may be about 50 µm to about 2000 µm. Thus, the thickness of the co-polymer may be about 50 µm, about 52 µm, about 54 µm, about 56 µm, about 58 µm, about 60 µm, about 62 µm, about 64 µm, about 66 µm, about 68 µm, about 70 µm, about 72 µm, about 74 µm, about 76 µm, about 78 µm, about 80 µm, about 82 µm, about 84 µm, about 86 µm, about 88 µm, about 90 µm, about 92 µm, about 94 µm, about 96 µm, about 98 µm, about 100 µm, about 110 µm, about 120 µm, about 130 µm, about 140 µm, about 150 µm, about 160 µm, about 170 µm, about 180 µm, about 190 µm, about 200 µm, about 220 µm, about 240 µm, about 260 µm, about 280 µm, about 300 µm, about 320 µm, about 340 µm, about 360 µm, about 380 µm, about 400 µm, about 420 µm, about 440 µm, about 460 µm, about 480 µm, about 500 µm, about 520 µm, about 540 µm, about 560 µm, about 580 µm, about 600 µm, about 620 µm, about 640 µm, about 660 µm, about 680 µm, about 700 µm, about 720 µm, about 740 µm, about 760 µm, about 780 µm, about 800 µm, about 820 µm, about 840 µm, about 860 µm, about 880 µm, about 900 µm, about 920 µm, about 940 µm, about 960 µm, about 980 µm, about 1000 µm, about 1050 µm, about 1100 µm, about 1150 µm, about 1200 µm, about 1250 µm, about 1300 µm, about 1350 µm, about 1400 µm, about 1450 µm, about 1500 µm, about 1550 µm, about 1600 µm, about 1650 µm, about 1700 µm, about 1750 µm, about 1800 µm, about 1850 µm, about 1900 µm, about 1950 µm, about 2000 µm, or any range including and/or in between any two of these values.

The co-polymer of the dressing composition may be provided by any suitable method known in the art. In any embodiment disclosed herein, the co-polymer may be formed *via* high dose rate irradiation. For example, copolymerization of polyvinylpyrrolidone (PVP) with citric acid using gamma irradiation may be achieved via the protocol described in Ajji, Z., Nucl. Instrum. Meth. B 265:179-182 (2007).

Irradiation may include, but are not limited to, e-beam irradiation, gamma irradiation, x-ray irradiation, or a combination of any two or more thereof. In any embodiment herein, the co-polymer may be formed *via* an irradiation dose rate of about 50 kGy/h to about 100 kGy/h; thus, the irradiation dose rate may be about 50 kGy/h, about 52 kGy/h, about 54 kGy/h, about 56 kGy/h, about 58 kGy/h, about 60 kGy/h, about 62 kGy/h, about 64 kGy/h, about 66 kGy/h, about 68 kGy/h, about 70 kGy/h, about 72 kGy/h, about 74 kGy/h, about 76 kGy/h, about 78 kGy/h, about 80 kGy/h, about 82 kGy/h, about 84 kGy/h, about 86 kGy/h, about 88 kGy/h, about 90 kGy/h, about 92 kGy/h, about 94 kGy/h, about 96 kGy/h, about 98 kGy/h, about 100 kGy/h, or any range including and/or in between any two of these values.

In any embodiment disclosed herein, the dressing composition may further include an absorbent material disposed adjacent to the hydrogel.

In any embodiment disclosed herein, the absorbent material may include a wound-facing side and an environmental-facing side. The wound-facing side of the absorbent material may be configured to be adjoined with the environmental-facing side of the co-polymer.

In any embodiment disclosed herein, the thickness of the absorbent material may be about 15 µm to about 500 µm. Thus, the thickness of the absorbent material may be about 15 µm, about 16 µm, about 17 µm, about 18 µm, about 19 µm, about 20 µm, about 22 µm, about 24 µm, about 26 µm, about 28 µm, about 30 µm, about 32 µm, about 34 µm, about 36 µm, about 38 µm, about 40 µm, about 42 µm, about 44 µm, about 46 µm, about 48 µm, about 50 µm, about 52 µm, about 54 µm, about 56 µm, about 58 µm, about 60 µm, about 62 µm, about 64 µm, about 66 µm, about 68 µm, about 70 µm, about 72 µm, about 74 µm, about 76 µm, about 78 µm, about 80 µm, about 82 µm, about 84 µm, about 86 µm, about 88 µm, about 90 µm, about 92 µm, about 94 µm, about 96 µm, about 98 µm, about 100 µm, about 110 µm, about 120 µm, about 130 µm, about 140 µm, about 150 µm, about 160 µm, about 170 µm, about 180 µm, about 190 µm, about 200 µm, about 220 µm, about 240 µm, about 260 µm, about 280 µm, about 300 µm, about 320 µm, about 340 µm, about 360 µm, about 380 µm, about 400 µm, about 420 µm, about 440 µm, about 460 µm, about 480 µm, about 500 µm, or any range including and/or in between any two of these values.

Additionally or alternatively, in some embodiments, the absorbent material may include a superabsorbent polymer, a non-woven carboxymethyl cellulose (CMC) pad, polyester, rayon, nylon, or a combination of any two or more thereof.

In any embodiment disclosed herein, the dressing composition may further include a backing layer. The backing layer may include a wound-facing side and an environmental-facing side. The wound facing side of the backing layer may be configured to be adjoined with the environmental-facing side of the absorbent material, and the wound-facing side of the absorbent material may be configured to be adjoined with the environmental-facing side of the co-polymer.

In any embodiment disclosed herein, the thickness of the backing layer may be about 10 µm to about 1000 µm. Thus, the thickness of the backing layer may be about 10 µm, about 11 µm, about 12 µm, about 13 µm, about 14 µm, about 15 µm, about 16 µm, about 17 µm, about 18 µm, about 19 µm, about 20 µm, about 22 µm, about 24 µm, about 26 µm, about 28 µm, about 30 µm, about 32 µm, about 34 µm, about 36 µm, about 38 µm, about 40 µm, about 42 µm, about 44 µm, about 46 µm, about 48 µm, about 50 µm, about 52 µm, about 54 µm, about 56 µm, about 58 µm, about 60 µm, about 62 µm, about 64 µm, about 66 µm, about 68 µm, about 70 µm, about 72 µm, about 74 µm, about 76 µm, about 78 µm, about 80 µm, about 82 µm, about 84 µm, about 86 µm, about 88 µm, about 90 µm, about 92 µm, about 94 µm, about 96 µm, about 98 µm, about 100 µm, about 110 µm, about 120 µm, about 130 µm, about 140 µm, about 150 µm, about 160 µm, about 170 µm, about 180 µm, about 190 µm, about 200 µm, about 220 µm, about 240 µm, about 260 µm, about 280 µm, about 300 µm, about 320 µm, about 340 µm, about 360 µm, about 380 µm, about 400 µm, about 420 µm, about 440 µm, about 460 µm, about 480 µm, about 500 µm, about 520 µm, about 540 µm, about 560 µm, about 580 µm, about 600 µm, about 620 µm, about 640 µm, about 660 µm, about 680 µm, about 700 µm, about 720 µm, about 740 µm, about 760 µm, about 780 µm, about 800 µm, about 820 µm, about 840 µm, about 860 µm, about 880 µm, about 900 µm, about 920 µm, about 940 µm, about 960 µm, about 980 µm, about 1000 µm, or any range including and/or in between any two of these values.

Additionally or alternatively, in some embodiments, the backing layer may include a polyurethane, a polyalkoxy alkyl acrylate, a polyalkoxy alkyl methacrylate, or a combination of any two or more thereof.

In any embodiment disclosed herein, the backing layer may be substantially impermeable to liquid and wound exudate. The backing layer may be configured to be impermeable to microorganisms. The backing layer may be configured to be semi-permeable to water vapor. In any embodiment disclosed herein, the backing layer may be configured to exhibit a moisture vapor transmission rate (MVTR) of about 300 g/m²/24hrs to about 20,000 g/m²/24hrs at 37.5°C at 50% relative humidity difference as described in ASTM E96-00. Thus, the backing layer may be configured to exhibit a MVTR of about 300 g/m²/24hrs, about 320 g/m²/24hrs, about 340 g/m²/24hrs, about 360 g/m²/24hrs, about 380 g/m²/24hrs, about 400 g/m²/24hrs, about 420 g/m²/24hrs, about 440 g/m²/24hrs, about 460 g/m²/24hrs, about 480 g/m²/24hrs, about 500 g/m²/24hrs, about 520 g/m²/24hrs, about 540 g/m²/24hrs, about 560 g/m²/24hrs, about 580 g/m²/24hrs, about 600 g/m²/24hrs, about 620 g/m²/24hrs, about 640 g/m²/24hrs, about 660 g/m²/24hrs, about 680 g/m²/24hrs, about 700 g/m²/24hrs, about 720 g/m²/24hrs, about 740 g/m²/24hrs, about 760 g/m²/24hrs, about 780 g/m²/24hrs, about 800 g/m²/24hrs, about 820 g/m²/24hrs, about 840 g/m²/24hrs, about 860 g/m²/24hrs, about 880 g/m²/24hrs, about 900 g/m²/24hrs, about 920 g/m²/24hrs, about 940 g/m²/24hrs, about 960 g/m²/24hrs, about 980 g/m²/24hrs, about 1000 g/m²/24hrs, about 1100 g/m²/24hrs, about 1200 g/m²/24hrs, about 1300 g/m²/24hrs, about 1400 g/m²/24hrs, about 1500 g/m²/24hrs, about 1600 g/m²/24hrs, about 1700 g/m²/24hrs, about 1800 g/m²/24hrs, about 1900 g/m²/24hrs, about 2000 g/m²/24hrs, about 2200 g/m²/24hrs, about 2400 g/m²/24hrs, about 2600 g/m²/24hrs, about 2800 g/m²/24hrs, about 3000 g/m²/24hrs, about 3200 g/m²/24hrs, about 3400 g/m²/24hrs, about 3600 g/m²/24hrs, about 3800 g/m²/24hrs, about 4000 g/m²/24hrs, about 4200 g/m²/24hrs, about 4400 g/m²/24hrs, about 4600 g/m²/24hrs, about 4800 g/m²/24hrs, about 5000 g/m²/24hrs, about 5200 g/m²/24hrs, about 5400 g/m²/24hrs, about 5600 g/m²/24hrs, about 5800 g/m²/24hrs, about 6000 g/m²/24hrs, about 6200 g/m²/24hrs, about 6400 g/m²/24hrs, about 6600 g/m²/24hrs, about 6800 g/m²/24hrs, about 7000 g/m²/24hrs, about 7200 g/m²/24hrs, about 7400 g/m²/24hrs, about 7600 g/m²/24hrs, about 7800 g/m²/24hrs, about 8000 g/m²/24hrs, about 8200 g/m²/24hrs, about 8400 g/m²/24hrs, about 8600 g/m²/24hrs, about 8800 g/m²/24hrs, about 9000 g/m²/24hrs, about 9200 g/m²/24hrs, about 9400 g/m²/24hrs, about 9600 g/m²/24hrs, about 9800 g/m²/24hrs, about 10000 g/m²/24hrs, about 10500 g/m²/24hrs, about 11000 g/m²/24hrs, about 11500 g/m²/24hrs, about 12000 g/m²/24hrs, about 12500 g/m²/24hrs, about 13000 g/m²/24hrs, about 13500 g/m²/24hrs, about 14000 g/m²/24hrs, about 14500 g/m²/24hrs, about 15000 g/m²/24hrs, about 15500 g/m²/24hrs, about 16000 g/m²/24hrs, about 16500 g/m²/24hrs, about 17000 g/m²/24hrs, about 17500 g/m²/24hrs, about 18000 g/m²/24hrs, about 18500 g/m²/24hrs, about 19000 g/m²/24hrs, about 19500 g/m²/24hrs, about 20000 g/m²/24hrs, or any range including and/or in between any two of the preceding values. Such moisture vapor transmission rates allow the wound under the wound dressing to heal under moist conditions without causing the skin surrounding the wound to macerate.

In any embodiment disclosed herein, the dressing composition may further include a bioresorbable layer comprising a mixture of a collagen and an oxidized regenerated cellulose (ORC).

In any embodiment disclosed herein, the collagen may include a type I collagen, a type II collagen, and/or a type III collagen. In any embodiment disclosed herein, the collagen may include mammalian collagen. In any embodiment disclosed herein, the collagen may include a human collagen. The human collagen may include human collagen type I and/or human collagen type III. In any embodiment disclosed herein, the collagen may include a bovine collagen, such as bovine collagen type I, bovine collagen type II, bovine collagen type III, and/or bovine collagen type IV. Collagen may be obtained from any natural source, may be chemically-modified collagen (e.g., an atelocollagen obtained by removing the immunogenic telopeptides from natural collagen), or may be any combination thereof. For example, the collagen may include collagen obtained from bovine corium that has been rendered largely free of non-collagenous components, for example, including fat, non-collagenous proteins, polysaccharides, and other carbohydrates, such as by procedures described in U.S. Pat. Nos. 4,614,794 and 4,320,201.

The collagen may be provided by any manner known in the art. For example, the collagen may be provided by a tissue sample and/or recombinantly manufactured. For example, human recombinant collagen may be provided via the protocol described in U.S. Pat. No. 5,962,648.

Further recombinant processes are set forth in U.S. Pat. No. 5,593,859 and WO2004/078120.

Collagen may be recombinantly manufactured by culturing a cell which has been transfected with at least one gene encoding a polypeptide that includes collagen. Collagen may be recombinantly manufactured by a plant (e.g., CollPlant, CollPlant Holdings Ltd., Ness Ziona, Israel) such as tobacco, or in yeast. Human recombinant collagen solution may be subsequently subjected to polymerization or cross-linking conditions to produce an insoluble fibrous collagen.

In any embodiment disclosed herein, the collagen may have a weight-average molecular weight of about 5,000 to about 100,000. Thus, the collagen may have a weight-average molecular weight of about 5,000, about 6,000, about 7,000, about 8,000, about 9,000, about 10,000, about 12,000, about 14,000, about 16,000, about 18,000, about 20,000, about 22,000, about 24,000, about 26,000, about 28,000, about 30,000, about 32,000, about 34,000, about 36,000, about 38,000, about 40,000, about 42,000, about 44,000, about 46,000, about 48,000, about 50,000, about 52,000, about 54,000, about 56,000, about 58,000, about 60,000, about 62,000, about 64,000, about 66,000, about 68,000, about 70,000, about 72,000, about 74,000, about 76,000, about 78,000, about 80,000, about 82,000, about 84,000, about 86,000, about 88,000, about 90,000, about 92,000, about 94,000, about 96,000, about 98,000, about 100,000, or any range including and/or in between any two of the preceding values.

In any embodiment disclosed herein, the bioresorbable layer may include about 0.1 wt.% to about 95 wt.% collagen. Thus, the amount of collagen in the bioresorbable layer may be about 0.1 wt.%, about 0.2 wt.%, about 0.3 wt.%, about 0.4 wt.%, about 0.5 wt.%, about 0.6 wt.%, about 0.7 wt.%, about 0.8 wt.%, about 0.9 wt.%, about 1 wt.%, about 1.1 wt.%, about 1.2 wt.%, about 1.3 wt.%, about 1.4 wt.%, about 1.5 wt.%, about 1.6 wt.%, about 1.7 wt.%, about 1.8 wt.%, about 1.9 wt.%, about 2 wt.%, about 2.2 wt.%, about 2.4 wt.%, about 2.6 wt.%, about 2.8 wt.%, about 3 wt.%, about 3.2 wt.%, about 3.4 wt.%, about 3.6 wt.%, about 3.8 wt.%, about 4 wt.%, about 4.2 wt.%, about 4.4 wt.%, about 4.6 wt.%, about 4.8 wt.%, about 5 wt.%, about 5.2 wt.%, about 5.4 wt.%, about 5.6 wt.%, about 5.8 wt.%, about 6 wt.%, about 6.2 wt.%, about 6.4 wt.%, about 6.6 wt.%, about 6.8 wt.%, about 7 wt.%, about 7.2 wt.%, about 7.4 wt.%, about 7.6 wt.%, about 7.8 wt.%, about 8 wt.%, about 8.2 wt.%, about 8.4 wt.%, about 8.6 wt.%, about 8.8 wt.%, about 9 wt.%, about 9.2 wt.%, about 9.4 wt.%, about 9.6 wt.%, about 9.8 wt.%, about 10 wt.%, about 11 wt.%, about 12 wt.%, about 13 wt.%, about 14 wt.%, about 15 wt.%, about 16 wt.%, about 17 wt.%, about 18 wt.%, about 19 wt.%, about 20 wt.%, about 22 wt.%, about 24 wt.%, about 26 wt.%, about 28 wt.%, about 30 wt.%, about 32 wt.%, about 34 wt.%, about 36 wt.%, about 38 wt.%, about 40 wt.%, about 42 wt.%, about 44 wt.%, about 46 wt.%, about 48 wt.%, about 50 wt.%, about 52 wt.%, about 54 wt.%, about 56 wt.%, about 58 wt.%, about 60 wt.%, about 62 wt.%, about 64 wt.%, about 66 wt.%, about 68 wt.%, about 70 wt.%, about 72 wt.%, about 74 wt.%, about 76 wt.%, about 78 wt.%, about 80 wt.%, about 82 wt.%, about 84 wt.%, about 86 wt.%, about 88 wt.%, about 90 wt.%, about 92 wt.%, about 94 wt.%, about 95 wt.%, or any range including and/or in between any two of these values.

In any embodiment disclosed herein, the collagen may include a weight ratio of human collagen type I to human collagen type III of about 100:0, about 90:10, about 80:20, about 70:30, about 60:40, about 50:50, about 40:60, about 30:70, about 20:80, about 10:90, about 0: 100, or any range including and/or in between any two of the preceding values.

In any embodiment disclosed herein, the oxidized regenerated cellulose (ORC) may be produced by the oxidation of cellulose, for example with dinitrogen tetroxide and/or as described in U.S. Pat. No. 3,122,479 (incorporated herein by reference). Without wishing to be bound by theory, it is believed that this process may convert primary alcohol groups on the saccharide residues of the cellulose to carboxylic acid groups, for example, forming uronic acid residues within the cellulose chain. The oxidation need not proceed with complete selectivity, and as a result hydroxyl groups on carbons 2 and 3 of the saccharide residue may be converted to the keto form. These ketone units may introduce an alkali labile link, which at pH 7 or higher initiates the decomposition of the polymer *via* formation of a lactone and sugar ring cleavage. As a result, oxidized regenerated cellulose is biodegradable and bioresorbable under physiological conditions. ORC is available with a variety of degrees of oxidation and hence rates of degradation.

In any embodiment disclosed herein, the bioresorbable layer may include about 30 wt.% to about 70 wt.% ORC with a weight-average molecular weight of about 10,000 to about 1,000,000. The bioresorbable layer may include ORC in an amount (by weight of the bioresorbable layer) of about 30 wt.%, about 32 wt.%, about 34 wt.%, about 36 wt.%, about 38 wt.%, about 40 wt.%, about 42 wt.%, about 44 wt.%, about 46 wt.%, about 48 wt.%, about 50 wt.%, about 52 wt.%, about 54 wt.%, about 56 wt.%, about 58 wt.%, about 60 wt.%, about 62 wt.%, about 64 wt.%, about 66 wt.%, about 68 wt.%, about 70 wt.%, or any range including and/or in between any two of the preceding values. The ORC have a weight-average molecular weight of about 10,000, about 11,000, about 12,000, about 13,000, about 14,000, about 15,000, about 16,000, about 17,000, about 18,000, about 19,000, about 20,000, about 22,000, about 24,000, about 26,000, about 28,000, about 30,000, about 32,000, about 34,000, about 36,000, about 38,000, about 40,000, about 42,000, about 44,000, about 46,000, about 48,000, about 50,000, about 55,000, about 60,000, about 65,000, about 70,000, about 75,000, about 80,000, about 85,000, about 90,000, about 95,000, about 100,000, about 110,000, about 120,000, about 130,000, about 140,000, about 150,000, about 160,000, about 170,000, about 180,000, about 190,000, about 200,000, about 210,000, about 220,000, about 230,000, about 240,000, about 250,000, about 260,000, about 270,000, about 280,000, about 290,000, about 300,000, about 310,000, about 320,000, about 330,000, about 340,000, about 350,000, about 360,000, about 370,000, about 380,000, about 390,000, about 400,000, about 410,000, about 420,000, about 430,000, about 440,000, about 450,000, about 460,000, about 470,000, about 480,000, about 490,000, about 500,000, about 510,000, about 520,000, about 530,000, about 540,000, about 550,000, about 560,000, about 570,000, about 580,000, about 590,000, about 600,000, about 610,000, about 620,000, about 630,000, about 640,000, about 650,000, about 660,000, about 670,000, about 680,000, about 690,000, about 700,000, about 710,000, about 720,000, about 730,000, about 740,000, about 750,000, about 760,000, about 770,000, about 780,000, about 790,000, about 800,000, about 810,000, about 820,000, about 830,000, about 840,000, about 850,000, about 860,000, about 870,000, about 880,000, about 890,000, about 900,000, about 910,000, about 920,000, about 930,000, about 940,000, about 950,000, about 960,000, about 970,000, about 980,000, about 990,000, about 1,000,000, or any range including and/or in between any two of these values.

The ORC may include particles, fibers, or both; in any embodiment disclosed herein, the ORC may be in the form of particles, such as fiber particles or powder particles. In embodiments that include ORC fibers, the ORC fibers may have a volume fraction such that at least 80% of the fibers have lengths in the range from about 5 µm to about 1,000 µm; thus, the ORC may include fiber lengths of about 5 µm, about 6 µm, about 7 µm, about 8 µm, about 9 µm, about 10 µm, about 11 µm, about 12 µm, about 13 µm, about 14 µm, about 15 µm, about 16 µm, about 17 µm, about 18 µm, about 19 µm, about 20 µm, about 22 µm, about 24 µm, about 26 µm, about 28 µm, about 30 µm, about 32 µm, about 34 µm, about 36 µm, about 38 µm, about 40 µm, about 42 µm, about 44 µm, about 46 µm, about 48 µm, about 50 µm, about 55 µm, about 60 µm, about 65 µm, about 70 µm, about 75 µm, about 80 µm, about 85 µm, about 90 µm, about 95 µm, about 100 µm, about 110 µm, about 120 µm, about 130 µm, about 140 µm, about 150 µm, about 160 µm, about 170 µm, about 180 µm, about 190 µm, about 200 µm, about 220 µm, about 230 µm, about 240 µm, about 250 µm, about 260 µm, about 280 µm, about 300 µm, about 320 µm, about 340 µm, about 360 µm, about 380 µm, about 400 µm, about 420 µm, about 440 µm, about 460 µm, about 480 µm, about 500 µm, about 550 µm, about 600 µm, about 650 µm, about 700 µm, about 750 µm, about 800 µm, about 850 µm, about 900 µm, about 950 µm, about 1,000 µm, or any range including and/or in between any two of these values.

The bioresorbable layer may include a weight ratio of collagen to ORC of about 90:10 to 10:90, such as from about 60:40 to about 40:60. The bioresorbable layer may include a weight ratio of collagen to ORC of about 90:10, about 85:15, about 80:20, about 75:25, about 70:30, about 65:35, about 60:40, about 55:45, about 50:50, about 45:55, about 40:60, about 35:65, about 30:70, about 25:75, about 20:80, about 15:85, about 10:90, or any range including and/or in between any two of these values.

Each of the hydrogel, the optional absorbent material, the optional backing layer, and the optional bioresorbable layer may independently include a wound-facing side and an environmental-facing side. In any embodiment disclosed herein, the wound-facing side of the backing layer may be configured to be adjoined with the environmental-facing side of the absorbent material, wherein the wound-facing side of the absorbent material may be configured to be adjoined with the environmental-facing side of the hydrogel, and wherein the wound-facing side of the hydrogel may be configured to be in contact with a wound when in use. In any embodiment disclosed herein, the wound-facing side of the backing layer may be configured to be adjoined with the environmental-facing side of the absorbent material, wherein the wound-facing side of the absorbent material may be configured to be adjoined with the environmental-facing side of the bioresorbable layer, wherein the wound-facing side of the bioresorbable layer may be configured to be adjoined to with the environmental-facing side of the hydrogel, and wherein the wound-facing side of the hydrogel may be configured to be in contact with a wound when in use.

In any embodiment disclosed herein, the dressing composition of the present technology may be sterile and packaged in a microorganism-impermeable container.

### Wound Dressing Compositions of the Present Technology

In a related aspect, the present technology provides a wound dressing composition that includes a mixture of a collagen of any embodiment disclosed herein, an oxidized regenerated cellulose (ORC) of any embodiment disclosed herein, and a co-polymer of any embodiment disclosed herein. In any embodiment disclosed herein, the wound dressing composition may include a wound-facing side and an environmental-facing side. The wound-facing side of the wound dressing composition may be configured to be in contact with a wound when in use.

The wound dressing composition may include about 0.1 wt.% to about 45 wt.% of the copolymer. Thus, the wound dressing composition may include about 0.1 wt.%, about 0.2 wt.%, about 0.3 wt.%, about 0.4 wt.%, about 0.5 wt.%, about 0.6 wt.%, about 0.7 wt.%, about 0.8 wt.%, about 0.9 wt.%, about 1 wt.%, about 1.1 wt.%, about 1.2 wt.%, about 1.3 wt.%, about 1.4 wt.%, about 1.5 wt.%, about 1.6 wt.%, about 1.7 wt.%, about 1.8 wt.%, about 1.9 wt.%, about 2 wt.%, about 2.2 wt.%, about 2.4 wt.%, about 2.6 wt.%, about 2.8 wt.%, about 3 wt.%, about 3.2 wt.%, about 3.4 wt.%, about 3.6 wt.%, about 3.8 wt.%, about 4 wt.%, about 4.2 wt.%, about 4.4 wt.%, about 4.6 wt.%, about 4.8 wt.%, about 5 wt.%, about 5.5 wt.%, about 6 wt.%, about 6.5 wt.%, about 7 wt.%, about 7.5 wt.%, about 8 wt.%, about 8.5 wt.%, about 9 wt.%, about 9.5 wt.%, about 10 wt.%, about 11 wt.%, about 12 wt.%, about 13 wt.%, about 14 wt.%, about 15 wt.%, about 16 wt.%, about 17 wt.%, about 18 wt.%, about 19 wt.%, about 20 wt.%, about 22 wt.%, about 24 wt.%, about 26 wt.%, about 28 wt.%, about 30 wt.%, about 32 wt.%, about 34 wt.%, about 36 wt.%, about 38 wt.%, about 40 wt.%, about 42 wt.%, about 44 wt.%, about 45 wt.%, or any range including and/or in between any two of these values.

In addition to the methods previously described for generating the co-polymer, the co-polymer of the wound dressing composition may be generated by drying a hydrogel of any embodiment described previously in this disclosure. The dried hydrogel may then be milled to provide a granular form of the co-polymer. In any embodiment disclosed herein, the dried hydrogel may be milled to provide a particle size less than 0.5 mm. Additionally or alternatively, in some embodiments, the dried hydrogel may be milled to provide a particle size of about 0.05 mm to about 0.5 mm. Thus, the dried hydrogel may be milled to provide a particle size of about 0.05 mm, about 0.06 mm, about 0.07 mm, about 0.08 mm, about 0.09 mm, about 0.1 mm, about 0.11 mm, about 0.12 mm, about 0.13 mm, about 0.14 mm, about 0.15 mm, about 0.16 mm, about 0.17 mm, about 0.18 mm, about 0.19 mm, about 0.2 mm, about 0.22 mm, about 0.24 mm, about 0.26 mm, about 0.28 mm, about 0.3 mm, about 0.32 mm, about 0.34 mm, about 0.36 mm, about 0.38 mm, about 0.4 mm, about 0.42 mm, about 0.44 mm, about 0.46 mm, about 0.48 mm, about 0.5 mm, or any range including and/or in between any two of these values.

In any embodiment disclosed herein, the wound dressing composition may include about 0.1 wt.% to about 95 wt.% collagen. Thus, the amount of collagen in the bioresorbable layer may be about 0.1 wt.%, about 0.2 wt.%, about 0.3 wt.%, about 0.4 wt.%, about 0.5 wt.%, about 0.6 wt.%, about 0.7 wt.%, about 0.8 wt.%, about 0.9 wt.%, about 1 wt.%, about 1.1 wt.%, about 1.2 wt.%, about 1.3 wt.%, about 1.4 wt.%, about 1.5 wt.%, about 1.6 wt.%, about 1.7 wt.%, about 1.8 wt.%, about 1.9 wt.%, about 2 wt.%, about 2.2 wt.%, about 2.4 wt.%, about 2.6 wt.%, about 2.8 wt.%, about 3 wt.%, about 3.2 wt.%, about 3.4 wt.%, about 3.6 wt.%, about 3.8 wt.%, about 4 wt.%, about 4.2 wt.%, about 4.4 wt.%, about 4.6 wt.%, about 4.8 wt.%, about 5 wt.%, about 5.2 wt.%, about 5.4 wt.%, about 5.6 wt.%, about 5.8 wt.%, about 6 wt.%, about 6.2 wt.%, about 6.4 wt.%, about 6.6 wt.%, about 6.8 wt.%, about 7 wt.%, about 7.2 wt.%, about 7.4 wt.%, about 7.6 wt.%, about 7.8 wt.%, about 8 wt.%, about 8.2 wt.%, about 8.4 wt.%, about 8.6 wt.%, about 8.8 wt.%, about 9 wt.%, about 9.2 wt.%, about 9.4 wt.%, about 9.6 wt.%, about 9.8 wt.%, about 10 wt.%, about 11 wt.%, about 12 wt.%, about 13 wt.%, about 14 wt.%, about 15 wt.%, about 16 wt.%, about 17 wt.%, about 18 wt.%, about 19 wt.%, about 20 wt.%, about 22 wt.%, about 24 wt.%, about 26 wt.%, about 28 wt.%, about 30 wt.%, about 32 wt.%, about 34 wt.%, about 36 wt.%, about 38 wt.%, about 40 wt.%, about 42 wt.%, about 44 wt.%, about 46 wt.%, about 48 wt.%, about 50 wt.%, about 52 wt.%, about 54 wt.%, about 56 wt.%, about 58 wt.%, about 60 wt.%, about 62 wt.%, about 64 wt.%, about 66 wt.%, about 68 wt.%, about 70 wt.%, about 72 wt.%, about 74 wt.%, about 76 wt.%, about 78 wt.%, about 80 wt.%, about 82 wt.%, about 84 wt.%, about 86 wt.%, about 88 wt.%, about 90 wt.%, about 92 wt.%, about 94 wt.%, about 95 wt.%, or any range including and/or in between any two of these values.

In any embodiment disclosed herein, the collagen may include a weight ratio of human collagen type I to human collagen type III of about 100:0, about 90:10, about 80:20, about 70:30, about 60:40, about 50:50, about 40:60, about 30:70, about 20:80, about 10:90, about 0: 100, or any range including and/or in between any two of the preceding values.

In any embodiment disclosed herein, the collagen may have a weight-average molecular weight of about 5,000 to about 100,000. Thus, the collagen may have a weight-average molecular weight of about 5,000, about 6,000, about 7,000, about 8,000, about 9,000, about 10,000, about 12,000, about 14,000, about 16,000, about 18,000, about 20,000, about 22,000, about 24,000, about 26,000, about 28,000, about 30,000, about 32,000, about 34,000, about 36,000, about 38,000, about 40,000, about 42,000, about 44,000, about 46,000, about 48,000, about 50,000, about 52,000, about 54,000, about 56,000, about 58,000, about 60,000, about 62,000, about 64,000, about 66,000, about 68,000, about 70,000, about 72,000, about 74,000, about 76,000, about 78,000, about 80,000, about 82,000, about 84,000, about 86,000, about 88,000, about 90,000, about 92,000, about 94,000, about 96,000, about 98,000, about 100,000, or any range including and/or in between any two of the preceding values.

In any embodiment disclosed herein, the wound dressing composition may include about 5 wt.% to about 70 wt.% ORC. The bioresorbable layer may include ORC in an amount (by weight of the bioresorbable layer) of about 5 wt.%, about 6 wt.%, about 7 wt.%, about 8 wt.%, about 9 wt.%, about 10 wt.%, about 11 wt.%, about 12 wt.%, about 13 wt.%, about 14 wt.%, about 15 wt.%, about 16 wt.%, about 17 wt.%, about 18 wt.%, about 19 wt.%, about 20 wt.%, about 22 wt.%, about 24 wt.%, about 26 wt.%, about 28 wt.%, about 30 wt.%, about 32 wt.%, about 34 wt.%, about 36 wt.%, about 38 wt.%, about 40 wt.%, about 42 wt.%, about 44 wt.%, about 46 wt.%, about 48 wt.%, about 50 wt.%, about 52 wt.%, about 54 wt.%, about 56 wt.%, about 58 wt.%, about 60 wt.%, about 62 wt.%, about 64 wt.%, about 66 wt.%, about 68 wt.%, about 70 wt.%, or any range including and/or in between any two of the preceding values.

In any embodiment disclosed herein, the ORC may have a weight-average molecular weight of about 10,000 to about 1,000,000. Thus, the ORC may have a weight-average molecular weight of about 10,000, about 11,000, about 12,000, about 13,000, about 14,000, about 15,000, about 16,000, about 17,000, about 18,000, about 19,000, about 20,000, about 22,000, about 24,000, about 26,000, about 28,000, about 30,000, about 32,000, about 34,000, about 36,000, about 38,000, about 40,000, about 42,000, about 44,000, about 46,000, about 48,000, about 50,000, about 55,000, about 60,000, about 65,000, about 70,000, about 75,000, about 80,000, about 85,000, about 90,000, about 95,000, about 100,000, about 110,000, about 120,000, about 130,000, about 140,000, about 150,000, about 160,000, about 170,000, about 180,000, about 190,000, about 200,000, about 210,000, about 220,000, about 230,000, about 240,000, about 250,000, about 260,000, about 270,000, about 280,000, about 290,000, about 300,000, about 310,000, about 320,000, about 330,000, about 340,000, about 350,000, about 360,000, about 370,000, about 380,000, about 390,000, about 400,000, about 410,000, about 420,000, about 430,000, about 440,000, about 450,000, about 460,000, about 470,000, about 480,000, about 490,000, about 500,000, about 510,000, about 520,000, about 530,000, about 540,000, about 550,000, about 560,000, about 570,000, about 580,000, about 590,000, about 600,000, about 610,000, about 620,000, about 630,000, about 640,000, about 650,000, about 660,000, about 670,000, about 680,000, about 690,000, about 700,000, about 710,000, about 720,000, about 730,000, about 740,000, about 750,000, about 760,000, about 770,000, about 780,000, about 790,000, about 800,000, about 810,000, about 820,000, about 830,000, about 840,000, about 850,000, about 860,000, about 870,000, about 880,000, about 890,000, about 900,000, about 910,000, about 920,000, about 930,000, about 940,000, about 950,000, about 960,000, about 970,000, about 980,000, about 990,000, about 1,000,000, or any range including and/or in between any two of these values.

The ORC may include particles, fibers, or both; in any embodiment disclosed herein, the ORC may be in the form of particles, such as fiber particles or powder particles. In embodiments that include ORC fibers, the ORC fibers may have a volume fraction such that at least 80% of the fibers have lengths in the range from about 5 µm to about 1,000 µm; thus, the ORC may include fiber lengths of about 5 µm, about 6 µm, about 7 µm, about 8 µm, about 9 µm, about 10 µm, about 11 µm, about 12 µm, about 13 µm, about 14 µm, about 15 µm, about 16 µm, about 17 µm, about 18 µm, about 19 µm, about 20 µm, about 22 µm, about 24 µm, about 26 µm, about 28 µm, about 30 µm, about 32 µm, about 34 µm, about 36 µm, about 38 µm, about 40 µm, about 42 µm, about 44 µm, about 46 µm, about 48 µm, about 50 µm, about 55 µm, about 60 µm, about 65 µm, about 70 µm, about 75 µm, about 80 µm, about 85 µm, about 90 µm, about 95 µm, about 100 µm, about 110 µm, about 120 µm, about 130 µm, about 140 µm, about 150 µm, about 160 µm, about 170 µm, about 180 µm, about 190 µm, about 200 µm, about 220 µm, about 230 µm, about 240 µm, about 250 µm, about 260 µm, about 280 µm, about 300 µm, about 320 µm, about 340 µm, about 360 µm, about 380 µm, about 400 µm, about 420 µm, about 440 µm, about 460 µm, about 480 µm, about 500 µm, about 550 µm, about 600 µm, about 650 µm, about 700 µm, about 750 µm, about 800 µm, about 850 µm, about 900 µm, about 950 µm, about 1,000 µm, or any range including and/or in between any two of these values.

The wound dressing composition may include a weight ratio of collagen to ORC of about 90:10 to 10:90, such as from about 60:40 to about 40:60. The bioresorbable layer may include a weight ratio of collagen to ORC of about 90:10, about 85:15, about 80:20, about 75:25, about 70:30, about 65:35, about 60:40, about 55:45, about 50:50, about 45:55, about 40:60, about 35:65, about 30:70, about 25:75, about 20:80, about 15:85, about 10:90, or any range including and/or in between any two of these values.

In any embodiment disclosed herein, the wound dressing composition may include a silver compound. The wound dressing composition may include about 0.1 wt.% to about 3 wt.% of a silver compound; thus, the silver compound of the wound dressing composition may be included in an amount of about 0.1 wt.%, about 0.11 wt.%, about 0.12 wt.%, about 0.13 wt.%, about 0.14 wt.%, about 0.15 wt.%, about 0.16 wt.%, about 0.17 wt.%, about 0.18 wt.%, about 0.19 wt.%, about 0.2 wt.%, about 0.22 wt.%, about 0.24 wt.%, about 0.26 wt.%, about 0.28 wt.%, about 0.3 wt.%, about 0.32 wt.%, about 0.34 wt.%, about 0.36 wt.%, about 0.38 wt.%, about 0.4 wt.%, about 0.42 wt.%, about 0.44 wt.%, about 0.46 wt.%, about 0.48 wt.%, about 0.50 wt.%, about 0.52 wt.%, about 0.54 wt.%, about 0.56 wt.%, about 0.58 wt.%, about 0.6 wt.%, about 0.62 wt.%, about 0.64 wt.%, about 0.66 wt.%, about 0.68 wt.%, about 0.7 wt.%, about 0.72 wt.%, about 0.74 wt.%, about 0.76 wt.%, about 0.78 wt.%, about 0.8 wt.%, about 0.82 wt.%, about 0.84 wt.%, about 0.86 wt.%, about 0.88 wt.%, about 0.9 wt.%, about 0.92 wt.%, about 0.94 wt.%, about 0.96 wt.%, about 0.98 wt.%, about 1 wt.%, about 1.1 wt.%, about 1.15 wt.%, about 1.2 wt.%, about 1.25 wt.%, about 1.3 wt.%, about 1.35 wt.%, about 1.4 wt.%, about 1.45 wt.%, about 1.5 wt.%, about 1.55 wt.%, about 1.6 wt.%, about 1.65 wt.%, about 1.7 wt.%, about 1.75 wt.%, about 1.8 wt.%, about 1.85 wt.%, about 1.9 wt.%, about 1.95 wt.%, about 2 wt.%, about 2.05 wt.%, about 2.1 wt.%, about 2.15 wt.%, about 2.2 wt.%, about 2.25 wt.%, about 2.3 wt.%, about 2.35 wt.%, about 2.4 wt.%, about 2.45 wt.%, about 2.5 wt.%, about 2.55 wt.%, about 2.6 wt.%, about 2.65 wt.%, about 2.7 wt.%, about 2.75 wt.%, about 2.8 wt.%, about 2.85 wt.%, about 2.9 wt.%, about 2.95 wt.%, about 3 wt.%, or any range including and/or in between any two of these values.

The silver compound of the wound dressing composition may include one or more pharmaceutically acceptable silver salts. Exemplary sources of the one or more pharmaceutically acceptable silver salts may include, but are not limited to, silver oxide, silver chromate, silver allantoinate, silver borate, silver glycerolate, silver nitrate, silver acetate, silver chloride, silver sulfate, silver lactate, silver bromide, silver iodide, silver carbonate, silver citrate, silver laurate, silver deoxycholate, silver salicylate, silver p-aminobenzoate, silver p- amino salicylate, nanocrystalline silver, or a combination of any two or more thereof. In any embodiment herin, at least a portion of any silver compound of the wound dressing composition of the present technology may be present as a complex of ORC with the silver compound *(e.g.,* an ORC-silver complex).

In any embodiment disclosed herein, the wound dressing composition may optionally include one or more additional biomaterials. The one or more additional biomaterials may be included in an amount of about 1 wt.% to about 25 wt.% of the wound dressing composition. Thus, the one or more additional biomaterials may be included in an amount of about 1 wt.%, about 2 wt.%, about 3 wt.%, about 4 wt.%, about 5 wt.%, about 6 wt.%, about 7 wt.%, about 8 wt.%, about 9 wt.%, about 10 wt.%, about 11 wt.%, about 12 wt.%, about 13 wt.%, about 14 wt.%, about 15 wt.%, about 16 wt.%, about 17 wt.%, about 18 wt.%, about 19 wt.%, about 20 wt.%, about 21 wt.%, about 22 wt.%, about 23 wt.%, about 24 wt.%, about 25 wt.%, or any range including and/or in between any two of these values. The one or more additional biomaterials may include gelatin, chitosan, fibronectin, hyaluronic acid, a polysaccharide, or a combination of any two or more thereof.

In any embodiment disclosed herein, the wound dressing composition may optionally include at least one plasticizer. The at least one plasticizer may be included in the wound dressing composition in an amount of about 1 wt.% to about 15 wt.%. Thus, the at least one plasticizer may be included in the wound dressing composition in an amount of about 1 wt.%, about 1.1 wt.%, about 1.2 wt.%, about 1.3 wt.%, about 1.4 wt.%, about 1.5 wt.%, about 1.6 wt.%, about 1.7 wt.%, about 1.8 wt.%, about 1.9 wt.%, about 2 wt.%, about 2.2 wt.%, about 2.4 wt.%, about 2.6 wt.%, about 2.8 wt.%, about 3 wt.%, about 3.2 wt.%, about 3.4 wt.%, about 3.6 wt.%, about 3.8 wt.%, about 4 wt.%, about 4.2 wt.%, about 4.4 wt.%, about 4.6 wt.%, about 4.8 wt.%, about 5 wt.%, about 5.2 wt.%, about 5.4 wt.%, about 5.6 wt.%, about 5.8 wt.%, about 6 wt.%, about 6.2 wt.%, about 6.4 wt.%, about 6.6 wt.%, about 6.8 wt.%, about 7 wt.%, about 7.2 wt.%, about 7.4 wt.%, about 7.6 wt.%, about 7.8 wt.%, about 8 wt.%, about 8.2 wt.%, about 8.4 wt.%, about 8.6 wt.%, about 8.8 wt.%, about 9 wt.%, about 9.2 wt.%, about 9.4 wt.%, about 9.6 wt.%, about 9.8 wt.%, about 10 wt.%, about 10.2 wt.%, about 10.4 wt.%, about 10.6 wt.%, about 10.8 wt.%, about 11 wt.%, about 11.2 wt.%, about 11.4 wt.%, about 11.6 wt.%, about 11.8 wt.%, about 12 wt.%, about 12.2 wt.%, about 12.4 wt.%, about 12.6 wt.%, about 12.8 wt.%, about 13 wt.%, about 13.2 wt.%, about 13.4 wt.%, about 13.6 wt.%, about 13.8 wt.%, about 14 wt.%, about 14.2 wt.%, about 14.4 wt.%, about 14.6 wt.%, about 14.8 wt.%, about 15 wt.%, or any range including and/or in between any two of the preceding values. Exemplary plasticizers include, but are not limited to, an acetylated monoglyceride, an alkyl citrate, methyl ricinoleate, glycerol, polyvinylpyrrolidone, or a combination of any two or more thereof. Examples of alkyl citrates include, but are not limited to, triethyl citrate, acetyl triethyl citrate, tributyl citrate, acetyl tributyl citrate, trioctyl citrate, acetyl trioctyl citrate, trihexyl citrate, acetyl trihexyl citrate, butyryl trihexyl citrate, trimethyl citrate, or a combination of any two or more thereof.

In any embodiment disclosed herein, a solid content of the wound dressing composition may be about 0.1 wt.% to about 10 wt.%; thus, the solid content of the wound dressing composition may be about 0.1 wt.%, about 0.11 wt.%, about 0.12 wt.%, about 0.13 wt.%, about 0.14 wt.%, about 0.15 wt.%, about 0.16 wt.%, about 0.17 wt.%, about 0.18 wt.%, about 0.19 wt.%, about 0.2 wt.%, about 0.22 wt.%, about 0.24 wt.%, about 0.26 wt.%, about 0.28 wt.%, about 0.3 wt.%, about 0.32 wt.%, about 0.34 wt.%, about 0.36 wt.%, about 0.38 wt.%, about 0.4 wt.%, about 0.42 wt.%, about 0.44 wt.%, about 0.46 wt.%, about 0.48 wt.%, about 0.5 wt.%, about 0.55 wt.%, about 0.6 wt.%, about 0.65 wt.%, about 0.7 wt.%, about 0.75 wt.%, about 0.8 wt.%, about 0.85 wt.%, about 0.9 wt.%, about 0.95 wt.%, about 1 wt.%, about 1.1 wt.%, about 1.2 wt.%, about 1.3 wt.%, about 1.4 wt.%, about 1.5 wt.%, about 1.6 wt.%, about 1.7 wt.%, about 1.8 wt.%, about 1.9 wt.%, about 2 wt.%, about 2.1 wt.%, about 2.2 wt.%, about 2.3 wt.%, about 2.4 wt.%, about 2.5 wt.%, about 2.6 wt.%, about 2.7 wt.%, about 2.8 wt.%, about 2.9 wt.%, about 3 wt.%, about 3.1 wt.%, about 3.2 wt.%, about 3.3 wt.%, about 3.4 wt.%, about 3.5 wt.%, about 3.6 wt.%, about 3.7 wt.%, about 3.8 wt.%, about 3.9 wt.%, about 4 wt.%, about 4.1 wt.%, about 4.2 wt.%, about 4.3 wt.%, about 4.4 wt.%, about 4.5 wt.%, about 4.6 wt.%, about 4.7 wt.%, about 4.8 wt.%, about 4.9 wt.%, about 5 wt.%, about 5.1 wt.%, about 5.2 wt.%, about 5.3 wt.%, about 5.4 wt.%, about 5.5 wt.%, about 5.6 wt.%, about 5.7 wt.%, about 5.8 wt.%, about 5.9 wt.%, about 6 wt.%, about 6.1 wt.%, about 6.2 wt.%, about 6.3 wt.%, about 6.4 wt.%, about 6.4 wt.%, about 6.6 wt.%, about 6.7 wt.%, about 6.8 wt.%, about 6.9 wt.%, about 7 wt.%, about 7.1 wt.%, about 7.2 wt.%, about 7.3 wt.%, about 7.4 wt.%, about 7.5 wt.%, about 7.6 wt.%, about 7.7 wt.%, about 7.8 wt.%, about 7.9 wt.%, about 8 wt.%, about 8.1 wt.%, about 8.2 wt.%, about 8.3 wt.%, about 8.4 wt.%, about 8.5 wt.%, about 8.6 wt.%, about 8.7 wt.%, about 8.8 wt.%, about 8.9 wt.%, about 9 wt.%, about 9.1 wt.%, about 9.2 wt.%, about 9.3 wt.%, about 9.4 wt.%, about 9.5 wt.%, about 9.6 wt.%, about 9.7 wt.%, about 9.8 wt.%, about 9.9 wt.%, about 10 wt.%, or any range including and/or in between any two of the preceding values.

In any embodiment disclosed herein, the wound dressing composition of the present technology may be sterile and packaged in a microorganism-impermeable container.

The wound dressing composition may be in the form of a freeze-dried sponge or a film material. In any embodiment disclosed herein, a suitable sponge may be generated by freeze-drying or solvent drying an aqueous dispersion of collagen, ORC, and co-polymer, similar to the protocols described in EP-A-11 53622.

In any embodiment disclosed herein, the wound dressing composition may be in the form of a freeze-dried sponge with an average pore size of about 10 to about 500 µm, such as about 100 to about 300 µm. Thus, the average pore size may be about 10 µm, about 12 µm, about 14 µm, about 16 µm, about 18 µm, about 20 µm, about 22 µm, about 24 µm, about 26 µm, about 28 µm, about 30 µm, about 32 µm, about 34 µm, about 36 µm, about 38 µm, about 40 µm, about 42 µm, about 44 µm, about 46 µm, about 48 µm, about 50 µm, about 52 µm, about 54 µm, about 56 µm, about 58 µm, about 60 µm, about 62 µm, about 64 µm, about 66 µm, about 68 µm, about 70 µm, about 72 µm, about 74 µm, about 76 µm, about 78 µm, about 80 µm, about 82 µm, about 84 µm, about 86 µm, about 88 µm, about 90 µm, about 92 µm, about 94 µm, about 96 µm, about 98 µm, about 100 µm, about 105 µm, about 110 µm, about 115 µm, about 120 µm, about 125 µm, about 130 µm, about 135 µm, about 140 µm, about 145 µm, about 150 µm, about 155 µm, about 160 µm, about 165 µm, about 170 µm, about 175 µm, about 180 µm, about 185 µm, about 190 µm, about 195 µm, about 200 µm, about 210 µm, about 220 µm, about 230 µm, about 240 µm, about 250 µm, about 260 µm, about 270 µm, about 280 µm, about 290 µm, about 300 µm, about 320 µm, about 340 µm, about 360 µm, about 380 µm, about 400 µm, about 420 µm, about 440 µm, about 460 µm, about 480 µm, about 500 µm, or any range including and/or in between any two of the preceding values.

In any embodiment disclosed herein, the dressing compositions and/or the wound dressing compositions (collectively referred to herein as "the dressings") of the present technology may be capable of preventing, reducing, inhibiting, or disrupting biofilm formation in a wound. Reducing a biofilm includes reducing the number of total viable microorganisms making up at least part of the biofilm, for example, as measured by total viable counts (TVC) of microorganisms (e.g., bacteria, yeast). The biofilm may comprise bacteria including, but not limited to *Pseudomonas aeruginosa*, *Staphylococcus aureus* and *Streptococcus mutans.* The biofilm may also include fungi including but not limited to yeasts, such as *Candida albicans.* Additionally or alternatively, the dressings of the present technology may be capable of preventing, reducing, inhibiting, or disrupting a biofilm in a wound by ≥ about 10% to ≥ about 100% after about 12 hours to about 24 hours *in vitro* exposure, or by ≥ about 1 log₁₀ units to by ≥ about 6 log₁₀ units after about 12 hours to about 24 hours *in vitro* exposure, compared to that observed in a wound of a control patient that does not receive the dressings of the present technology. Additionally or alternatively, the dressings of the present technology may be capable of preventing, reducing, inhibiting, or disrupting a biofilm in a wound by ≥ about 10%, ≥ about 15%, ≥ about 20%, ≥ about 25%, ≥ about 30%, ≥ about 35%, ≥ about 40%, ≥ about 45%, ≥ about 50%, ≥ about 55%, ≥ about 60%, ≥ about 65%, ≥ about 70%, ≥ about 75%, ≥ about 80%, ≥ about 85%, ≥ about 90%, ≥ about 95%, ≥ about 99%, ≥ about 100%, or any range including and/or in between any two of the preceding values, compared to that observed in a wound of a control patient that does not receive the dressings of the present technology. Additionally or alternatively, the dressings of the present technology may be capable of preventing, reducing, inhibiting, or disrupting a biofilm in a wound by ≥ about 1 log₁₀ units, by ≥ about 1.5 log₁₀ units, by ≥ about 2 log₁₀ units, by ≥ about 2.5 log₁₀ units, by ≥ about 3 log₁₀ units, by ≥ about 3.5 log₁₀ units, by ≥ about 4 log₁₀ units, by ≥ about 4.5 log₁₀ units, by ≥ about 5 log₁₀ units, by ≥ about 5.5 log₁₀ units, by ≥ about 6 log₁₀ units, or any range including and/or in between any two of the preceding values, compared to that observed in a wound of a control patient that does not receive the dressings of the present technology.

The therapeutic efficacy of the dressings of the present technology may be assayed using any method known to those in the art. An exemplary method to test the therapeutic efficacy of the dressings of the present technology is the colony drip flow reactor (C-DFR) assay (*see* Lipp, C., et al., J. Wound Care 19:220-226(2010)).

### Therapeutic and Prophylactic Methods of the Present Technology

In an aspect, the present disclosure provides a method for treating a wound in a subject in need thereof, wherein the method comprises administering to the wound one or more dressings of any embodiment disclosed herein. The wound may be an acute wound or a chronic wound. The wound may be an acute wound, such as a burn, a skin graft, and/or a dehisced surgical wound. The wound may be a chronic wound, such as an infectious wound, a venous ulcer, an arterial ulcer, a decubitis ulcer, and/or a diabetic ulcer. The dressing may protect the wound from infection, such as a bacterial infection (e.g., caused by gram-negative and/or gram-positive bacteria) or a fungal infection.

Examples of gram-positive bacteria include, but are not limited to *Actinomyces* sp., *Arcanobacterium* sp., *Bacillus* sp., *Bavariicoccus* sp., *Brachybacterium* sp., *Clostridium* sp., *Cnuibacter* sp., *Corynebacterium* sp., *Enterococcus* sp., *Desulfitobacterium* sp., *Fervidobacterium* sp., *Georgenia* sp., *Janibacter* sp., *Lactobacillales* sp., *Microbispora* sp., *Nocardia* sp., *Pasteuria* sp., *Pilibacter* sp., *Propionibacterium* sp., *Rathayibacter* sp., *Rhodococcus* sp., *Roseburia* sp., *Rothia* sp., *Sarcina* sp., *Solibacillus* sp., *Sporosarcina* sp., *Staphylococcus* sp., *Streptococcus* sp., *Syntrophomonas* sp., or *Tepidibacter* sp.

Examples of gram-negative bacteria include, but are not limited to *Acetobacter* sp., *Acidaminococcus* sp., *Acinetobacter* sp., *Agrobacterium* sp., *Akkermansia* sp., *Anaerobiospirillum* sp., *Anaerolinea* sp., *Arcobacter* sp., *Armatimonas* sp., *Azotobacter* sp., *Bacteroides* sp., *Bacteroidetes* sp., *Bartonella* sp., *Bdellovibriosp., Brachyspira* sp., *Bradyrhizobium* sp., *Caldilinea* sp., *Cardiobacterium* sp., *Christensenella* sp., *Chthonomonas* sp., *Coxiella* sp., *Cyanobacteria* sp., *Cytophaga* sp., *Dehalogenimonas* sp., *Desulfurobacterium* sp., *Devosia* sp., *Dialister* sp., *Dictyoglomus* sp., *Dinoroseobacter* sp., *Enterobacter* sp., *Escherichia* sp., *Fimbriimonas* sp., *Flavobacterium* sp., *Francisella* sp., *Fusobacterium* sp., *Gluconacetobacter* sp., *Haemophilus* sp., *Helicobacter* sp., *Kingella* sp., *Klebsiella sp., Kluyvera* sp., *Kozakia* sp., *Legionella* sp. *Leptonema* sp. *Leptotrichia* sp., *Levilinea* sp. *Luteimonas* sp. *Megamonas* sp., *Megasphaera* sp., *Meiothermus* sp., *Methylobacterium* sp., *Moraxella* sp., *Morganella* sp., *Mycoplasma* sp., *Neisseria* sp., *Nitrosomonas* sp., *Pectinatus* sp., *Pedobacter* sp., *Pelosinus* sp., *Propionispora* sp., *Proteus* sp., *Pseudomonas* sp., *Pseudoxanthomonas* sp., *Rickettsia* sp., *Salinibacter* sp., *Salmonella* sp., *Samsonia* sp., *Serratia* sp., *Shigella* sp., *Shimwellia* sp., *Sphingomonas* sp., *Stenotrophomonas* sp., *Thorselliaceae* sp., *Vampirococcus* sp., *Verminephrobacter* sp., *Vibrio* sp., *Victivallis* sp., *Vitreoscilla* sp., and *Wolbachia* sp.

Fungal infections may be caused by *Aspergillus* sp., *Aureobasidium* sp., *Candida* sp., *Cladosporium* sp., *Curvularia* sp., *Engodontium* sp., *Epicoccum* sp., *Gibberella* sp., *Hypocreales* sp., *Leptosphaerulina* sp., *Malessezia* sp., *Penicillium* sp., *Rhodosporidium* sp., *Trichosporon* sp., *Trichtophyton* sp., and/or *Ulocladium* sp.

In any embodiment herein, the wound may include a biofilm and the dressings of the present technology prevent, reduce, inhibit, and/or disrupt the biofilm.

In another aspect, the present disclosure provides a method for maintaining reduced biofilm levels in a wound in a subject in need thereof, wherein the method includes administering to the wound one or more dressings of any embodiment disclosed herein. The wound may be an acute wound or a chronic wound. The wound may be an acute wound, such as a burn, a skin graft, and/or a dehisced surgical wound. The wound may be a chronic wound, such as an infectious wound, a venous ulcer, an arterial ulcer, a decubitis ulcer, and/or a diabetic ulcer. The dressing may protect the wound from infection, such as a bacterial infection (e.g., caused by gram-negative and/or gram-positive bacteria) or a fungal infection.

Any method known to those in the art for administering dressings to an acute or a chronic wound disclosed herein may be employed, but the method of treatment by therapy are not part of the present invention. Suitable methods include *in vitro* or *in vivo* methods. *In vivo* methods typically include the administration of one or more dressings to a subject in need thereof, suitably a human. When used *in vivo* for therapy, the one or more dressings described herein are administered to the subject in effective amounts (*i.e.*, amounts that have desired therapeutic effect). The dose and dosage regimen will depend upon the state of the wound of the subject, and the characteristics of the particular dressing used.

The effective amount may be determined during pre-clinical trials and clinical trials by methods familiar to physicians and clinicians. An effective amount of one or more dressings useful in the methods may be administered to a subject in need thereof by any number of well-known methods for administering dressings.

### Methods of Making the Wound Dressings of the Present Technology

The wound dressings of the present technology may be produced by adding a collagen (either in paste or powder form) to an acidic solution (e.g., about 0.05 M acetic acid or other soluble organic acids), which may result in a final solution with 1.1 g solid collagen weight per 100 mL acid solution. The collagen will then be blended with the acid solution and allowed to swell to produce a homogenous slurry, at which point the ORC is blended into the collagen slurry (0.7 g ORC per 100 mL slurry) along with powdered PVP-CA material (0.2 g PVP-CA per 100 mL slurry) to generate a slurry material with about 2% solid content. Additionally or alternatively, in some embodiments, a slurry material may be generated with a solid content of about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9%, about 5%, about 5.1%, about 5.2%, about 5.3%, about 5.4%, about 5.5%, about 5.6%, about 5.7%, about 5.8%, about 5.9%, about 6%, about 6.1%, about 6.2%, about 6.3%, about 6.4%, about 6.5%, about 6.6%, about 6.7%, about 6.8%, about 6.9%, about 7%, about 7.1%, about 7.2%, about 7.3%, about 7.4%, about 7.5%, about 7.6%, about 7.7%, about 7.8%, about 7.9%, about 8%, about 8.1%, about 8.2%, about 8.3%, about 8.4%, about 8.5%, about 8.6%, about 8.7%, about 8.8%, about 8.9%, about 9%, about 9.1%, about 9.2%, about 9.3%, about 9.4%, about 9.5%, about 9.6%, about 9.7%, about 9.8%, about 9.9%, about 10%, or any range including and/or in between any two of these values. The resulting slurry is then degassed using a vacuum to remove trapped air and dispensed into a container before being flash frozen in a -70°C freezer to form a block. Additionally or alternatively, in some embodiments, the resulting slurry may be flash frozen in a freezer at a temperature of about -70°C, about -65°C, about -60°C, about - 55°C, about -50°C, about -45°C, about -40°C, about -35°C, about -30°C, about -25°C, about - 20°C, or any range including and/or in between any two of these values. The resulting block of collagen/ORC/PVP-CA may then freeze dried to produce a bioresorbable sponge material, which may be further trimmed to produce wound dressing compositions of different thicknesses.

### Kits Comprising the Dressings of the Present Technology

In a further related aspect, the present disclosure provides kits that include a dressing of any embodiment described herein and instructions for use. The instructions may describe performing any method described herein and any embodiment described herein of such methods. The kit may optionally comprise components such as antiseptic wipes, ointment, adhesive tape, tweezers, and/or scissors.

### EQUIVALENTS

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third, and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

## Claims

1. A wound dressing composition comprising a hydrogel, wherein the hydrogel comprises a co-polymer of polyvinylpyrrolidone (PVP) and citric acid.

2. The wound dressing composition of claim 1, wherein the hydrogel comprises about 5 % (w/v) to about 25 % (w/v) co-polymer.

3. The wound dressing composition of claim 1 or claim 2, wherein the hydrogel has a thickness of about 50 µm to about 2000 µm.

4. The wound dressing composition of any one of claims 1-3, wherein the hydrogel comprises a wound-facing side and an environmental facing side.

5. The wound dressing composition of any one of claims 1-4, further comprising an absorbent material disposed adjacent to the hydrogel, optionally wherein the absorbent material comprises a superabsorbent polymer, a non-woven carboxymethyl cellulose (CMC) pad, polyester, rayon, nylon, or a combination of any two or more thereof.

6. The wound dressing composition of any one of claims 1-5, further comprising a backing layer.

7. The wound dressing composition of claim 6, wherein the backing layer comprises a polyurethane, a polyalkoxy alkyl acrylate, a polyalkoxy alkyl methacrylate, or a combination of any two or more thereof.

8. The wound dressing composition of claim 6 or claim 7, wherein the thickness of the backing layer has a thickness of about 10 µm to about 1000 µm.

9. The wound dressing composition of any one of claims 6-8, wherein the backing layer is configured to exhibit a moisture vapor transmission rate (MVTR) of about 300 g/m²/24hrs to about 20,000 g/m²/24hrs.

10. The wound dressing composition of any one of claims 1-9, wherein the wound dressing comprises
a first layer comprising the hydrogel; and
a second layer comprising a mixture of a collagen and an oxidized regenerated cellulose (ORC).

11. The wound dressing composition of claim 10, wherein the second layer comprises about 30 wt.% to about 70 wt.% ORC and about 0.1 wt.% to about 60 wt.% collagen.

12. The wound dressing composition of claim 10 or claim 11, wherein the ORC of the second layer has a weight-average molecular weight of about 10,000 to about 1,000,000.

13. The wound dressing composition of any one of claims 10-12, wherein the ORC of the second layer comprises fiber lengths of about 5 µm to about 1,000 µm.

14. The wound dressing composition of any one of claims 10-13, wherein the collagen of the second layer is a mammalian collagen.

15. The wound dressing composition of any one of claims 10-14, wherein the collagen comprises a bovine collagen, a human collagen, a recombinantly derived collagen, and a combination of any two or more thereof.

16. The wound dressing composition of any one of claims 10-15, wherein the collagen of the second layer has a weight-average molecular weight of about 5,000 to about 100,000.

17. The wound dressing composition of claim 1, wherein the co-polymer of polyvinylpyrrolidone (PVP) and citric acid is mixed with collagen and an oxidized regenerated cellulose (ORC).

18. The wound dressing composition of claim 17, wherein the co-polymer comprises a weight ratio of PVP to citric acid of about 25:1 to about 1:2.

19. The wound dressing composition of claim 17 or claim 18, wherein the wound dressing composition comprises about 0.1 wt.% to about 45 wt.% of the copolymer.

20. The wound dressing composition of any one of claims 17-19, wherein the wound dressing composition comprises about 30 wt.% to about 70 wt.% ORC.

21. The wound dressing composition of any one of claims 17-20, wherein the collagen comprises a mammalian collagen.

22. The wound dressing composition of any one of claims 17-21, wherein the collagen comprises a bovine collagen, a human collagen, a recombinantly derived collagen, or a combination of any two or more thereof.

23. The wound dressing composition of any one of claims 17-22, wherein the wound dressing composition comprises about 0.1 wt.% to about 60 wt.% collagen.

24. The wound dressing composition of any one of claims 17-23, wherein the collagen has a weight-average molecular weight of about 5,000 to about 100,000.

## Patentansprüche

1. Eine Wundverbandzusammensetzung, aufweisend ein Hydrogel, wobei das Hydrogel ein Copolymer aus Polyvinylpyrrolidon (PVP) und Zitronensäure aufweist.

2. Die Wundverbandzusammensetzung nach Anspruch 1, wobei das Hydrogel zu etwa 5 % (w/v) bis etwa 25 % (w/v) das Copolymer aufweist.

3. Die Wundverbandzusammensetzung nach Anspruch 1 oder 2, wobei das Hydrogel eine Dicke von etwa 50 µm bis etwa 2000 µm hat.

4. Die Wundverbandzusammensetzung nach einem der Ansprüche 1 bis 3, wobei das Hydrogel eine der Wunde zugewandte Seite und eine der Umgebung zugewandte Seite aufweist.

5. Die Wundverbandzusammensetzung nach einem der Ansprüche 1 bis 4, ferner aufweisend ein angrenzend an das Hydrogel angeordnetes absorbierendes Material, optional wobei das absorbierende Material ein superabsorbierendes Polymer, ein Carboxymethylcellulosevlieskissen (CMC-Vlieskissen), Polyester, Reyon, Nylon oder eine Kombination aus zwei oder mehr davon aufweist.

6. Die Wundverbandzusammensetzung nach einem der Ansprüche 1 bis 5, ferner aufweisend eine Trägerschicht.

7. Die Wundverbandzusammensetzung nach Anspruch 6, wobei die Trägerschicht ein Polyurethan, ein Polyalkoxyalkylacrylat, ein Polyalkoxyalkylmethacrylat oder eine Kombination aus zwei oder mehr davon aufweist.

8. Die Wundverbandzusammensetzung nach Anspruch 6 oder 7, wobei die Dicke der Trägerschicht eine Dicke von etwa 10 µm bis etwa 1000 µm hat.

9. Die Wundverbandzusammensetzung nach einem der Ansprüche 6 bis 8, wobei die Trägerschicht konfiguriert ist, um eine Dampfdurchlässigkeitszahl (MVTR) von etwa 300 g/m²/24 Std. bis etwa 20.000 g/m²/24 Std vorzuweisen.

10. Die Wundverbandzusammensetzung nach einem der Ansprüche 1 bis 9, wobei der Wundverband aufweist:
eine erste Schicht, aufweisend das Hydrogel; und
eine zweite Schicht, aufweisend eine Mischung aus Kollagen und einer oxidierten regenerierten Zellulose (ORC).

11. Die Wundverbandzusammensetzung nach Anspruch 10, wobei die zweite Schicht zu etwa 30 Gew.-% bis etwa 70 Gew.-% ORC und zu etwa 0,1 Gew.-% bis etwa 60 Gew.-% Kollagen aufweist.

12. Die Wundverbandzusammensetzung nach Anspruch 10 oder 11, wobei die ORC der zweiten Schicht ein gewichtsmittleres Molekulargewicht von etwa 10.000 bis etwa 1.000.000 hat.

13. Die Wundverbandzusammensetzung nach einem der Ansprüche 10 bis 12, wobei die ORC der zweiten Schicht Faserlängen von etwa 5 µm bis etwa 1.000 µm aufweist.

14. Die Wundverbandzusammensetzung nach einem der Ansprüche 10 bis 13, wobei das Kollagen der zweiten Schicht ein Säugetierkollagen ist.

15. Die Wundverbandzusammensetzung nach einem der Ansprüche 10 bis 14, wobei das Kollagen ein Rinderkollagen, ein menschliches Kollagen, ein rekombinant gewonnenes Kollagen und eine Kombination aus zwei oder mehr davon aufweist.

16. Die Wundverbandzusammensetzung nach einem der Ansprüche 10 bis 15, wobei das Kollagen der zweiten Schicht ein gewichtsmittleres Molekulargewicht von etwa 5.000 bis etwa 100.000 hat.

17. Die Wundverbandzusammensetzung nach Anspruch 1, wobei das Copolymer aus Polyvinylpyrrolidon (PVP) und Zitronensäure mit Kollagen und einer oxidierten regenerierten Zellulose (ORC) gemischt ist.

18. Die Wundverbandzusammensetzung nach Anspruch 17, wobei das Copolymer ein Gewichtsverhältnis von PVP zu Zitronensäure von etwa 25 : 1 bis etwa 1 - 2 aufweist.

19. Die Wundverbandzusammensetzung nach Anspruch 17 oder 18, wobei die Wundverbandzusammensetzung zu etwa 0,1 Gew.-% bis etwa 45 Gew.-% das Copolymer aufweist.

20. Die Wundverbandzusammensetzung nach einem der Ansprüche 17 bis 19, wobei die Wundverbandzusammensetzung zu etwa 30 Gew.-% bis etwa 70 Gew.-% ORC aufweist.

21. Die Wundverbandzusammensetzung nach einem der Ansprüche 17 bis 20, wobei das Kollagen ein Säugetierkollagen aufweist.

22. Die Wundverbandzusammensetzung nach einem der Ansprüche 17 bis 21, wobei das Kollagen ein Rinderkollagen, ein menschliches Kollagen, ein rekombinant gewonnenes Kollagen oder eine Kombination aus zwei oder mehr davon aufweist.

23. Die Wundverbandzusammensetzung nach einem der Ansprüche 17 bis 22, wobei die Wundverbandzusammensetzung zu etwa 0,1 Gew.-% bis etwa 60 Gew.-% Kollagen aufweist.

24. Die Wundverbandzusammensetzung nach einem der Ansprüche 17 bis 23, wobei das Kollagen ein gewichtsmittleres Molekulargewicht von etwa 5.000 bis etwa 100.000 hat.

## Revendications

1. Composition de pansement pour blessure comprenant un hydrogel, dans laquelle l'hydrogel comprend un copolymère de polyvinylpyrrolidone (PVP) et d'acide citrique.

2. Composition de pansement pour blessure selon la revendication 1, dans laquelle l'hydrogel comprend environ 5 % (p/v) à environ 25 % (p/v) de copolymère.

3. Composition de pansement pour blessure selon la revendication 1 ou la revendication 2, dans laquelle l'hydrogel a une épaisseur d'environ 50 µm à environ 2 000 µm.

4. Composition de pansement pour blessure selon l'une quelconque des revendications 1-3, dans laquelle l'hydrogel comprend une face orientée vers la blessure et une face orientée vers l'environnement.

5. Composition de pansement pour blessure selon l'une quelconque des revendications 1-4, comprenant en outre un matériau absorbant disposé adjacent à l'hydrogel, éventuellement dans laquelle le matériau absorbant comprend un polymère superabsorbant, un tampon de carboxyméthylcellulose (CMC) non tissé, du polyester, de la rayonne, du nylon, ou une combinaison de deux ou plusieurs quelconques de ceux-ci.

6. Composition de pansement pour blessure selon l'une quelconque des revendications 1-5, comprenant en outre une couche de support.

7. Composition de pansement pour blessure selon la revendication 6, dans laquelle la couche de support comprend un polyuréthane, un polyalkoxy-alkyl acrylate, un polyalkoxy-alkyl méthacrylate, ou une combinaison de deux ou plusieurs quelconque de ceux-ci.

8. Composition de pansement pour blessure selon la revendication 6 ou la revendication 7, dans laquelle l'épaisseur de la couche de support a une épaisseur d'environ 10 µm à environ 1 000 µm.

9. Composition de pansement pour blessure selon l'une quelconque des revendications 6-8, dans laquelle la couche de support est conçue pour présenter un taux de perméabilité à la vapeur d'eau (MVTR) d'environ 300 g/m²/24 heures à environ 20 000 g/m²/24 heures.

10. Composition de pansement pour blessure selon l'une quelconque des revendications 1-9, dans laquelle le pansement pour blessure comprend
une première couche comprenant l'hydrogel ; et
une seconde couche comprenant un mélange d'un collagène et d'une cellulose régénérée oxydée (ORC).

11. Composition de pansement pour blessure selon la revendication 10, dans laquelle la seconde couche comprend environ 30 % en poids à environ 70 % en poids d'ORC et environ 0,1 % en poids à environ 60 % en poids de collagène.

12. Composition de pansement pour blessure selon la revendication 10 ou la revendication 11, dans laquelle l'ORC de la seconde couche a un poids moléculaire moyen d'environ 10 000 à environ 1 000 000.

13. Composition de pansement pour blessure selon l'une quelconque des revendications 10-12, dans laquelle l'ORC de la seconde couche comprend des longueurs de fibres d'environ 5 µm à environ 1 000 µm.

14. Composition de pansement pour blessure selon l'une quelconque des revendications 10-13, dans laquelle le collagène de la seconde couche est un collagène de mammifère.

15. Composition de pansement pour blessure selon l'une quelconque des revendications 10-14, dans laquelle le collagène comprend un collagène bovin, un collagène humain, un collagène dérivé par recombinaison, et une combinaison de deux ou plusieurs quelconque de ceux-ci.

16. Composition de pansement pour blessure selon l'une quelconque des revendications 10-15, dans laquelle le collagène de la seconde couche a un poids moléculaire moyen d'environ 5 000 à environ 100 000.

17. Composition de pansement pour blessure selon la revendication 1, dans laquelle le copolymère de polyvinylpyrrolidone (PVP) et d'acide citrique est mélangé avec du collagène et une cellulose régénérée oxydée (ORC).

18. Composition de pansement pour blessure selon la revendication 17, dans laquelle le copolymère comprend un rapport de poids de PVP/acide citrique d'environ 25:1 à environ 1:2.

19. Composition de pansement pour blessure selon la revendication 17 ou la revendication 18, dans laquelle la composition de pansement pour blessure comprend environ 0,1 % en poids à environ 45 % en poids du copolymère.

20. Composition de pansement pour blessure selon l'une quelconque des revendications 17-19, dans laquelle la composition de pansement pour blessure comprend environ 30 % en poids à environ 70 % en poids d'ORC.

21. Composition de pansement selon l'une quelconque des revendications 17-20, dans laquelle le collagène comprend un collagène de mammifère.

22. Composition de pansement pour blessure selon l'une quelconque des revendications 17-21, dans laquelle le collagène comprend un collagène bovin, un collagène humain, un collagène dérivé par recombinaison, ou une combinaison de deux ou plusieurs quelconque de ceux-ci.

23. Composition de pansement pour blessure selon l'une quelconque des revendications 17-22, dans laquelle la composition de pansement pour blessure comprend environ 0,1 % en poids à environ 60 % en poids de collagène.

24. Composition de pansement pour blessure selon l'une quelconque des revendications 17-23, dans laquelle le collagène a un poids moléculaire moyen d'environ 5 000 à environ 100 000.
